# EUROPEAN PATENT SPECIFICATION

(11) **EP 3 242 944 B1**
(45) Date of publication and mention of the grant of the patent: **02.11.2022**
(21) Application number: 16707948.2
(22) Date of filing: 07.01.2016
(51) Int. Cl.: C12N 15/82, A01H 5/00

(54) **ALLELIC VARIANT OF THE CYC-B GENE FOR THE CONSTITUTION OF TOMATO VARIETIES RESISTANT TO BROOMRAPE**
ALLELISCHE VARIANTE DES CYC-B-GENS ZUR ZUSAMMENSETZUNG VON GEGEN SOMMERWURZ RESISTENTEN TOMATENVARIETÄTEN
VARIANT ALLÉLIQUE DU GÈNE CYC-B POUR LA CONSTITUTION DE VARIÉTÉS DE TOMATE RÉSISTANTES À L'OROBRANCHE

(30) Priority: 09.01.2015 IT MI20150009
(43) Date of publication of application: 15.11.2017
(73) Proprietor: Agenzia Lucana Di Sviluppo E Di Innovazione In Agricoltura (Alsia), 75100 Matera (MT) (IT)
(72) Inventor: CARRIERO, Filomena, 75024 Montescaglioso (MT) (IT); CELLINI, Francesco, 75100 Matera (MT) (IT)
(74) Representative: Calogero, Ida
(86) International application number: PCT/IB2016/050060
(87) International publication number: WO 2016/110814

(56) References cited:
- WO-A2-2006/098626
- MARIA FRANCESCA SILLETTI ET AL: "An increase of lycopene content in tomato fruit is associated with a novel Cyc-B allele isolated through TILLING technology", MOLECULAR BREEDING, vol. 31, no. 3, 22 December 2012 (2012-12-22), pages 665-674, XP055203233, ISSN: 1380-3743, DOI: 10.1007/s11032-012-9824-6
- RONEN G ET AL: "An alternative pathway to beta-carotene formation in plant chromoplasts discovered by map-based cloning of Beta and old-gold color mutations in tomato", PROCEEDINGS OF THE NATIONAL ACADEMY OF SCIENCES, NATIONAL ACADEMY OF SCIENCES, US, vol. 97, no. 20, 26 September 2000 (2000-09-26), pages 11102-11107, XP002310093, ISSN: 0027-8424, DOI: 10.1073/PNAS.190177497 cited in the application & DATABASE EMBL [Online] 9 October 2000 (2000-10-09), "Lycopersicon esculentum chromoplast-specific lycopene beta-cyclase mRNA, complete cds.", retrieved from EBI accession no. EM_STD:AF254793 Database accession no. AF254793
- MCCALLUM C M ET AL: "Targeting Induced Local Lesions IN Genomes (TILLING) for Plant Functional Genomics", PLANT PHYSIOLOGY, AMERICAN SOCIETY OF PLANT PHYSIOLOGISTS, ROCKVILLE, MD, US, vol. 123, no. 2, 1 June 2000 (2000-06-01), pages 439-442, XP002995773, ISSN: 0032-0889, DOI: 10.1104/PP.123.2.439 cited in the application
- WOUTER KOHLEN ET AL: "The tomato CAROTENOID CLEAVAGE DIOXYGENASE8 ( SlCCD8 ) regulates rhizosphere signaling, plant architecture and affects reproductive development through strigolactone biosynthesis", NEW PHYTOLOGIST, vol. 196, no. 2, 1 October 2012 (2012-10-01), pages 535-547, XP055203214, ISSN: 0028-646X, DOI: 10.1111/j.1469-8137.2012.04265.x cited in the application
- EVGENIA DOR ET AL: "Characterization of a novel tomato mutant resistant to the weedy parasites Orobanche and Phelipanche spp", EUPHYTICA, KLUWER ACADEMIC PUBLISHERS, DO, vol. 171, no. 3, 23 September 2009 (2009-09-23), pages 371-380, XP019768744, ISSN: 1573-5060 cited in the application
- JONATHAN T. VOGEL ET AL: "SlCCD7 controls strigolactone biosynthesis, shoot branching and mycorrhiza-induced apocarotenoid formation in tomato", THE PLANT JOURNAL, vol. 61, no. 2, 18 November 2009 (2009-11-18), pages 300-311, XP055340401, GB ISSN: 0960-7412, DOI: 10.1111/j.1365-313X.2009.04056.x

## Description

The present invention relates to an allelic variant of the gene Cyc-B, induced artificially, for the constitution of tomato varieties resistant to broomrape and the tomato varieties thus obtained.

Broomrapes are weeds, lacking chlorophyll and a root system, which take nutrients and water at the expense of the host plant they are parasitizing. They belong to the family of Orobanchacecae and over 100 species are included in the genre Orobanche, among which branching broomrape which prefers solanaceous plants and is the most widespread species on the Italian territory.

The presence of branching broomrape has increased significantly in recent years and its growing diffusion in areas dedicated to the cultivation of industrial tomatoes has produced considerable damage from both a quantitative and qualitative point of view [1].

Parasitized tomato plants initially show a more or less stunted growth followed by a quali-quanti-tative decrease in production, due to a reduction in the capacity of availing of nutrients and water absorption. The decreasing production is extremely variable as it depends on the duration of the parasitization: if it occurs from the first growth phases of the tomato, it is obviously greater, reaching up to about 40% and in some cases even up to 75% with respect to the production obtainable without infestation [2].

The harmfulness of this plant is mainly due to its capacity of rapidly infesting the land, reproducing itself exponentially through the production of extremely high quantities of seeds (up to 500,000 per plant) [3] having small dimensions (about 0.2-0.3 mm), whose vitality in the earth can last for even twelve years [4] . Due to their dimensions, the seeds are very easily scattered by the wind, water, animals and human activity using agricultural tools and machines and especially through the mechanical harvesting of tomatoes which is effected by cutting the plants to ground level where there are also stems of Broomrape ([5]-[7]). The ripe capsules release seeds, some of which remain dormant whereas others, if stimulated by root exudates of the host plants, are capable of germinating immediately and creating new plants that emerge from the ground forming floral scapes (shoots).

There is considerable interest in solving the problem of broomrape due to the damage caused by the parasite at an economic level in the agricultural field, the processing industry and consequently also for the consumer.

Numerous approaches have been used for controlling the infection, consisting in the use of herbicides, solarization, disinfecting the soil, rotations with plants traps, tillage, nitrogen fertilizers, manuring, biocontrol agents and secondary metabolites administered directly to the ground or in activated composts ([6], [8]) but none of these has proved to be completely effective.

Research conducted on site on control methods applied individually have provided only partial or completely unsatisfactory results, due to the variability of the climatic conditions of the different environments ([9], [11]). The integrated use of the above methods can limit damage [12] with the aim of progressively reducing the "seed bank" of the parasite in the ground to a minimum threshold level [13].

An alternative and/or complementary approach to that currently used for controlling infection by broomrape in tomatoes is genetic improvement or the selection and use of new genotypes of tomatoes capable of opposing the attack of the parasite.

β-carotene is not only a precursor of β-xanthophylls but is also a synthesis substrate of strigolactone, a hormone that exerts various functions in the life cycle of the plant [14].

Strigolactone is mainly produced in the roots and can in fact be found in both the exudates and also in the root tissue extracts of vegetable species belonging to monocotyledons and dicotyledons ([15]-[18]).

At a root level, strigolactone acts as a signal for the development of mycorrhizae [19] and as a stimulant for the germination of broomrape, a known parasite of numerous vegetable species [20], among which tomatoes.

The involvement of strigolactone in the susceptibility of tomato plants to broomrape infections is confirmed by various studies in which it is demonstrated that tomato lines with a low content of strigolactone are less subject to broomrape infections.

As already mentioned, strigolactone derives from β-carotene and specifically its synthesis substrate is the 9-cis-β-carotene isomer.

In tomatoes, the synthesis of β-carotene is catalyzed by two enzymes: lycopene beta cyclase, Lcy-b, encoded by the gene CrtL-b [21], which is expressed and active in the leaves, flowers and fruit and lycopene beta cyclase, Cyc-B, encoded by the gene Cyc-B, chromoplast specific, highly expressed in the petals and also present, at low levels, in the fruit [22].

International patent application WO2006/098626 describes a method for the production of plants, also tomatoes, resistant to infection on the part of Broomrape by the silencing or suppression of the genes belonging to the pathway of carotenoids, citing *inter alia* phytoene synthase, phytoene desaturase, carotene desaturase, isomerase carotene, lycopene cyclase, β-carotene hydroxylase, zeaxanthin epoxidase, neoxanthin synthase, carotenoid dioxygenase, 9-cis-epoxide carotenoid dioxygenase, cytochrome P450 hydroxylase, epoxidase, dehydrogenase and demethylase.

In the work of Kohlen W. et al., 2012 [23], in which the expression level of the gene CCD8 that encodes an enzyme involved in the synthesis of strigolactone, is modified through the antisense strategy, it is specified that transgenic tomato lines with low levels of the transcript CCD8 produce low quantities of strigolactone and have a 90% reduction in infection on the part of broomrape with respect to their counterparts (control plants) in which the expression of the gene CCD8 has not been modified.

The work of Vogel T. J. et al., 2010 describes tomato plants obtained through the antisense approach against the target gene CCD7, which are definitely more resistant to infection on the part of broomrape, but which parallelly have undesired features for breeding.

In both cases, in fact, an increase in the branchings of tomato plants generated with the antisense strategy is associated with a reduction in strigolactone caused by the deactivation of the two genes CCD7 and CCD8 belonging to the CCD family (carotenoid cleavage dioxygenase). Furthermore, in the work of Kohlen W. et al., 2012, in addition to an increase in the branchings, it is indicated that the deactivation of the gene CCD8 also has important consequences on the reproductive system, on the size of the flowers, on the quantity of seeds, and therefore on the fertility of the antisense plants thus obtained.

The mutant tomato *S1-ORT1,* generated by physical mutagenesis and identified by its resistance to infestations of broomrape [24] upon biochemical analysis of the root extracts, has proved to produce small quantities of the two strigolactones present in tomatoes, solanacol and didehydro-orobanchol, with respect to its strict control *M82* [25]. In addition to the "positive" feature of resistance to infestations on the part of broomrape, the tomato mutant *S1-ORT1,* on a vegetative level, showed, on the other hand, an increase in lateral branchings with respect to the control. This confirms the role of strigolactone in regulating the architecture of the plant and that it functions on a vegetative level as lateral branching suppressor. The gene responsible for the broomrape parasite-resistant feature, in *S1-ORT1,* is however still unknown.

New technologies have been developed in support of traditional technologies in the last decade, in order to increase the efficiency of the selection process of features of interest.

TILLING (Targeting Induced Local Lesions In Genomes) is a**mong** these new genetic improvement technologies.

TILLING is a method which combines chemical mutagenesis, already widely used in the past in the genetic improvement of various crops, and advanced biological knowledge and techniques for isolating new allelic variants (mutations) and therefore new genotypes that can be inserted, quite rapidly, in varietal constitution programs [26].

From a technological point of view, TILLING is a platform that develops a collection of genotypes that can be rapidly analyzed for selecting features of interest, by means of high-efficiency DNA analysis.

Using this technology, the authors of the present invention have now artificially induced and isolated an allelic variant of the gene which encodes the enzyme lycopene beta-cyclase (Cyc-B) responsible for the conversion of lycopene to β-carotene in the carotenoid pathway capable of conferring to tomato plants, the phenotype of resistance to the broomrape parasite, leaving the morphology and functionality of the reproductive system of the plant unaltered. In particular, the tomato mutant thus obtained does not have an increase in lateral branchings with respect to the control, overcoming undesired effects on the development of the plant of the mutants so far known (*Sl-ORT1;* CCD8, CCD7). Furthermore, no negative consequence on the reproductive system of the plant has been observed in terms of a reduction in the dimensions of the flowers, or the dimensions and number of seeds per tomato fruit. These effects are evidently completely unexpected considering that the inactivation of other genes of the carotenoid pathway leads to an increase in the branchings and influences the reproductive system and therefore the efficiency of the breeding process.

An object of the present invention therefore relates to the use of the allelic variant of the gene encoding the enzyme lycopene beta-cyclase (Cyc-B) (GenBank accession no. AF 254793) characterized by the presence of the missense mutation in position A949G, induced through mutagenesis to select a mutated tomato plant with a phenotype of resistance to infections by broomrapes, in particular branching broomrape, wherein said allelic variant consists in the nucleotide sequence (SEQ ID NO:5).

The allelic variant is characterized by the presence of a point mutation in position 949 of the sequence encoding the gene Cyc-B (A949G) which, at the protein level, causes an amino-acid change in position 317, more specifically *the* basic amino-acid Arginine (R) is substituted by the hydrophilic amino-acid Glycine (G), (R317G) .

From a comparative analysis with sequences deposited in data banks, the allelic variant A949G of the gene Cyc-B proves not to be present in the natural variability of tomato plants.

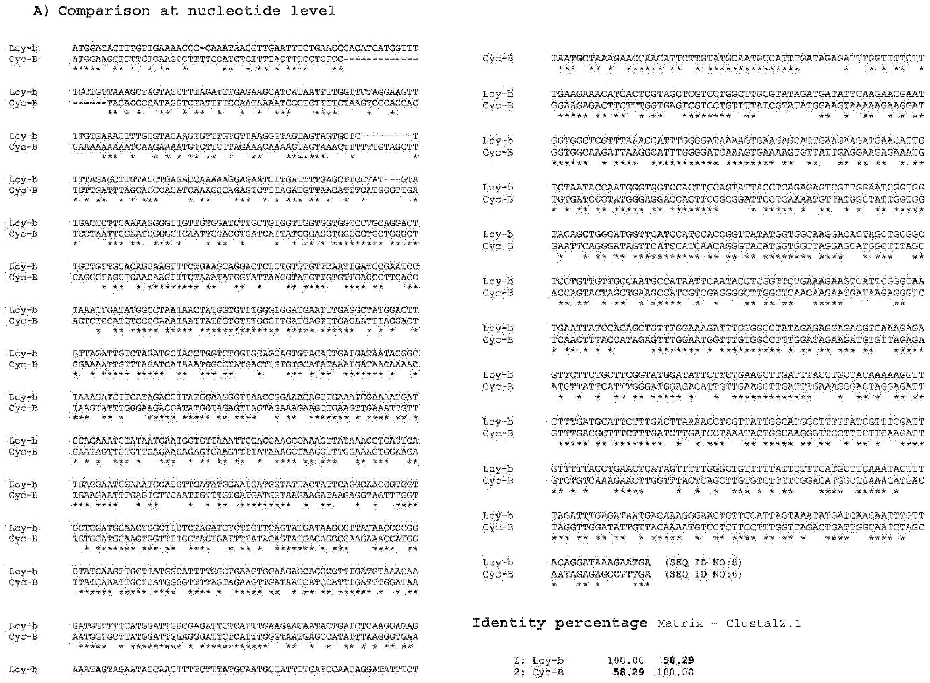

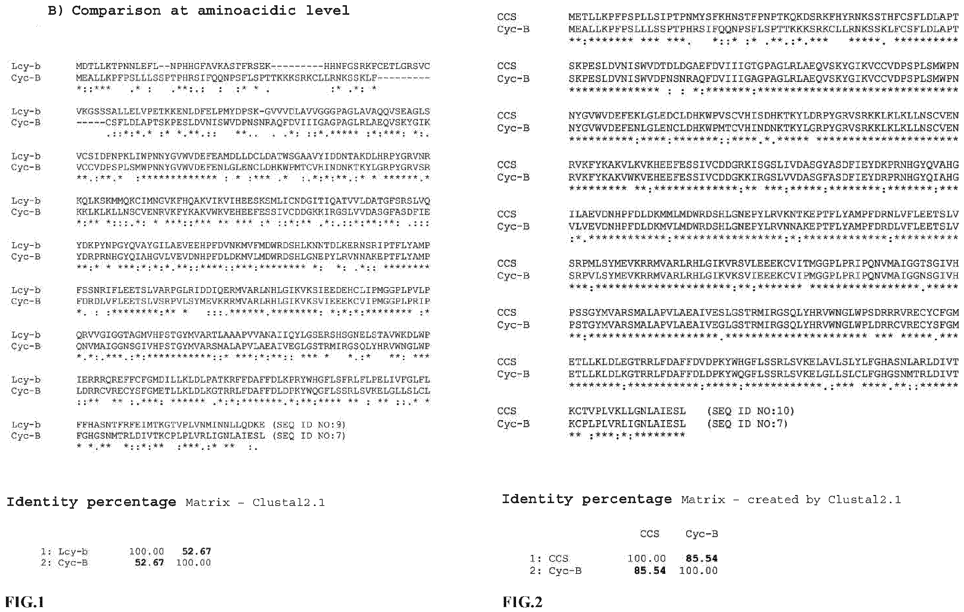

The nucleotide and wild-type amino-acid sequences of the specific lycopene cyclase chromoplast (Cyc-B) of *Solanum lycopersicum* (SEQ ID NO:6 and SEQ ID NO 7) have a sequence identity at a nucleotide and amino-acid level equal to only 58% and 52% with respect to the corresponding wild-type sequences of the lycopene cyclase (Lcy-b) of *Solanum lycopersicum* (GenBank access number X86452.1, SEQ ID NO:8 and UniProtKB/Swiss-Prot:Q43503, SEQ ID NO:9) . Figure 1 illustrates the comparison between the sequences at a nucleotide and amino-acid level and the relative identity percentages.

In this respect, it should be noted that the lycopene cyclase Cyc-B of *Solanum lycopersicum* (SEQ ID NO:7) has a much higher identity percentage at an amino-acid level (about 85%) with the enzyme capsanthin/capsorubin synthase (CCS) of peppers (UniProtKB-Q42435, SEQ ID NO:10), which, although it is an enzyme of the carotenoid pathway, is not a lycopene cyclase (see comparison illustrated in Figure 2) .

At a phenotype level, the mutation A949G of the gene Cyc-B, present in the tomato mutant line cyc-b7, causes an increase in lycopene to the detriment of β-carotene [30] .

On the basis of the hypothesis that the reduction in the production of β-carotene in the tomato mutant cyc-b7 can cause a reduction of strigolactone and consequently the plants can have a lower susceptibility to infection on the part of broomrape, the allelic variant of Cyc-B having the missense mutation A949G, induced artificially through mutagenesis, was assessed for two consecutive years in open fields for its susceptibility to the attack of the broomrape parasite. In both trials, effected in 2013 and 2014 in the area of Foggia where the problem of broomrape is strongly felt, it showed a good resistance to the attack of branching broomrape. In productive terms, the cyc-b7 mutant line produced 10% more fruit with respect to the control line (see Example 2). Finally, the invention relates to a screening method for identifying a tomato plant resistant to broomrape, said method comprising the analysis of a DNA sample from a tomato plant of interest for the presence of at least one missense mutation in position A949G of the gene Cyc-B consisting in SEQ ID NO:5.

The present invention will now be described, for illustrative but non-limiting purposes, according to some preferred embodiments with particular reference to the enclosed figures, in which:
- Figures 1A and 1B show a comparison at a nucleotide and amino-acid level between the wild-type sequences of lycopene cyclase Cyc-B (SEQ ID NOs:6-7) and Lyc-b (SEQ ID NOs:8-9) of *Solanum lycopersicum;*
- Figure 2 shows a comparison at an amino-acid level between the wild-type sequences of capsanthin/ capsorubin synthase (CCS) of peppers (SEQ ID NO:10) of *Solanum lycopersicum;*
- Figure 3 shows the structure of the gene Cyc-B produced with the use of the PARSESNP program (http://www.proweb.org/parsesnp/). The position of the fragments of PCR used for the molecular analysis is indicated together with their respective length. The two pairs of specific primers are represented by triangles: the external primers indicated with F1 and R1 and the internal primers indicated with F2 and R2;
- Figure 4 shows the nucleotide sequence of the allelic variant A949G of the gene Cyc-B. The point mutation induced artificially through chemical mutagenesis is indicated in grey and in bold print. The start codons (ATG) and transduction codons STOP (TGA) are indicated in the sequence in bold print;
- Figure 5 shows the photographs of the mutant plants cyc-b7 and Red Setter control plants in the field test of 2014. The dry shoots of broomrape which are much more abundant in the lot of the control plant Red Setter, can be seen in the photographs;
- Figure 6 shows the difference in the number of shoots observed between the lot hosting the mutant plants cyc-b7 and that of the control line Red Setter. The photographs were taken after the removal of the plants. EXAMPLE 1: *Isolation of the new allelic variant A949G of the gene Cyc-B*

### MATERIALS AND METHODS

### TILLING platform of Red Setter tomato

The TILLING platform of the tomato plant of Alsia-Centro Ricerche Metapontum Agrobios was used for isolating the new allelic variant of the gene Cyc-B [27]. This platform is based on a tomato mutant population generated in the genetic background of cv Red Setter, an industrial tomato variety. The platform comprises 5221 samples of DNA prepared from the same number of families M3 of the collection of Red Setter mutants.

### Projecting and selection of the primers

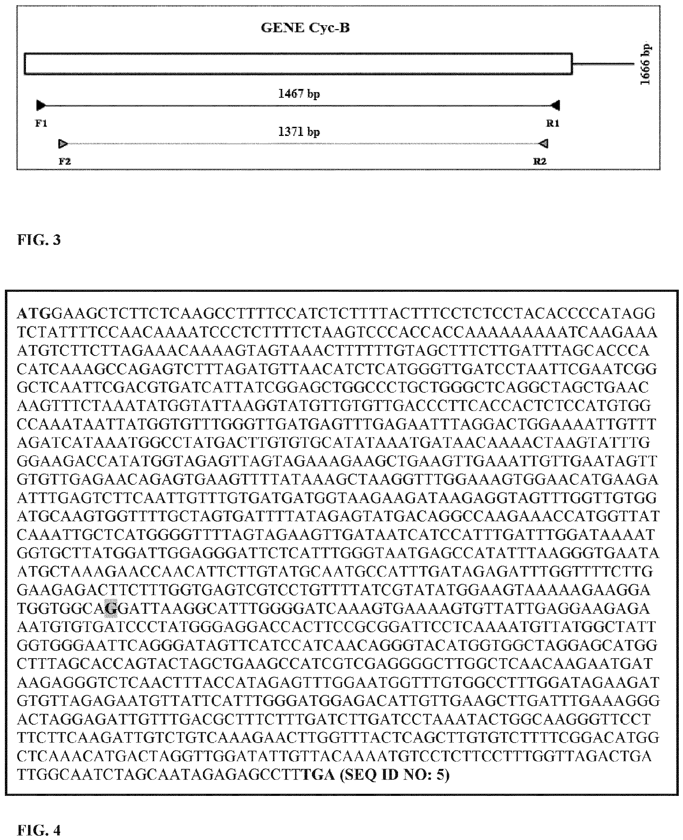

The complete sequence of the gene Cyc-B (GenBank accession no. AF 254793) 1666 bp long, consisting of 169 bp of 3'UTR and 1497 bp of ORF (Open Reading Frame) that encodes a polypeptide of 498 amino acids (Figure 3), was recovered in NCBI (National Center for Biotechnology Information; http://www.ncb.nlm.nih.gov/) data banks. The gene Cyc-B is a single-copy gene and does not have introns.

Two pairs of gene-specific oligonucleotides used in nested-PCR for the TILLING screening were designed on the basis of the sequence: a pair of external oligonucleotides that produce an amplification of 1467 bp and a pair of internal oligonucleotides marked at 5' with the fluorophores IRDye 700 (primer forward) and IRDye 800 (primer reverse) that amplify a fragment of 1371 bp.

The two pairs of primers were designed using the algorithm Primer3, available online at the site http://www.frodo.wi.mit.edu/primer3/ [28].

Table 1 indicates the sequences of the primers, the annealing temperatures of the pairs of primers and the dimensions of the amplicons used in the TILLING screening of the gene Cyc-B.

**TABLE 1**

| **Primer** | **Sequence** | **T°C** | **Length amplified** |
|---|---|---|---|
| *PCR1* | | 58°C | 1467 bp |
| F1 | 5' GCTCTTCTCAAGCCTTTTCC 3' (SEQ ID NO: 1) | | |
| R1 | 5' GCCAAT CAGT CT AACCAAAGG 3' (SEQ ID NO: 2) | | |
| *PCR2* | | 57°C | 1371 bp |
| F2 | 5' CCTCTCCTACACCCCATAGGT 3' (SEQ ID NO:3) | | |
| R2 | 5' AGCCATGTCCGAAAAGACAC 3' (SEQ ID NO:4) | | |

The portion of the gene Cyc-B selected for the search for mutations is shown in Figure 3.

### Molecular analyses

The search for useful mutations was effected by means of nested-PCR. For this purpose, the pair of external primers was used, called F1 (primer forward) and R1 (primer reverse) for effecting the first amplification (PCR1)and the pair of internal primers, called F2 (primer forward) and R2 (primer reverse) selected so as to amplify the product of PCR1, both described in Table 1.

Table 1 indicates the sequences of the primers, the annealing temperatures, the pairs, and the dimensions of the amplicons used in the TILLING screening of the gene Cyc-B. Both pairs of oligonucleotides were tested on genomic DNA of cv Red Setter and on various samples of the TILLING platform in order to define the optimal amplification conditions.

After establishing the experimental conditions of the nested-PCR and above all, after verifying that the product obtained with the two pairs of primers consisting of a single DNA band, the internal primers F2 and R2 were then acquired, respectively marked at the 5'terminal end with IRDye 700 and IRDye 800 that absorb and emit fluorescence in the near-infrared region. The marking of the internal primers is necessary for visualizing mutations with a LI-COR DNA Analyzer 4300 (LI-COR Inc.2006; http://www.licor.com).

All the oligonucleotides were supplied by the company Eurofins MWG/Operon (Eberserg, Germany). For PCR1, in a volume of 25 µl, 4 ng of genomic DNA were used as template for the amplification of a reaction mixture containing 10 pmoles of primer F1 and R1, 5 mM of dNTP, 10 mM Tris-HCl (pH 8.8), 1,5 mM MgCl₂, 50 mM KCl, 0.1% Triton X-100 and 1 Unit of DNA polymerase DyNAzyme (Finnzymes). After a first denaturing cycle at 94°C for 3', the amplification product was produced with 35 cycles each comprising a denaturing step at 94°C, annealing at 58°C and synthesis at 72°C for a time equal to 30" for each step. 1 µl of PCR1 was used as template for the amplification in PCR2 in the presence of 5 pmoles of marked primers F2 and R2 and under the same experimental conditions as PCR1 except that a temperature of 57°C was used for the annealing.

The PCR product thus obtained was subjected to a denaturing and re-association phase for the formation of hybrid DNA molecules consisting of wild-type filament and a muted filament in the presence of the mutation in the region analyzed. The point mutations were identified with the use of the endonuclease ENDO I (Serial Genetics) which recognizes the mismatched regions of the heteroduplex DNA molecules. The digestion conditions are those indicated by Triques et al., 2007 [29]. The digestion products were separated on acrylamide gel under denaturing conditions using the fragment analyzer Li-Cor 4300. The TIFF images in "grayscale" of the channels 700 and 800, as also the image in RGB, which allows the contemporaneous visualization of the two fluorochromes, were analyzed using Adobe Photoshop software for the search for point mutations.

### Sequence Analysis

The mutation identified through the screening of the TILLING platform was verified by means of sequence analysis.

The template for the sequence reaction was prepared by means of nested-PCR using the two pairs of primers adopted for the molecular screening of the TILLING. In this case, however, non-marked primers were used for the PCR2.

About 15-20 ng of nested-PCR product was used for the sequence reaction effected in a total volume of 20 µl using the Big Dye Terminator V.3.1 Kit (Applied Biosystem, Forster City, Ca, USA) and the protocol provided by the distributor of the kit. After purification on Sephadex G50 Fine columns, effected for eliminating the nucleotides and the primers not incorporated during the reaction, the sequence products were analyzed with the automatic capillary sequencer 3130 (Applera) and the chromatograms examined for the presence of mutation.

### RESULTS

The oligonucleotides selected and specific for the gene *Cyc-B* were used for the molecular screening of the tomato TILLING platform developed at the laboratories of Alsia-Centro Ricerche Metapontum Agrobios.

The analysis of the 5,221 samples of DNA organized in pools of 8 allowed 9 point mutations to be identified in eight mutant lines of tomato, as two nucleotide substitutions were found in the same genotype (Silletti et al. 2013). The identity and exact position of the nucleotide substitutions was verified by means of sequence analysis. Of the nine mutations, three proved to be silent whereas six at a protein level caused an amino-acid change.

### Results of the phenotyping analysis and selection of genotypes of interest

Of the six missense mutations identified, three were subjected to phenotyping analysis in order to study the effect of the amino-acid change on the phenotype.

Among these, the allelic variant that, in position 949 of the sequence encoding the gene *Cyc-B* (Figure 4) has a guanine instead of adenine (A949G), showed a modified phenotype with respect to the controls used in the phenotyping analysis [30].

The nucleotide sequence of the allelic variant A949G of Cyc-B is the following:

ATGGAAGCTCTTCTCAAGCCTTTTCCATCTCTTTTACTTTCCTCTCCTACACCCC

ATAGGTCTATTTTCCAACAAAATCCCTCTTTTCTAAGTCCCACCACCAAAAAAA

ATCAAGAAAATGTCTTCTTAGAAACAAAAGTAGTAAACTTTTTTGTAGCTTTCTT

GATTTAGCACCCACATCAAAGCCAGAGTCTTTAGATGTTAACATCTCATGGGTTG

ATCCTAATTCGAATCGGGCTCAATTCGACGTGATCATTATCGGAGCTGGCCCTGC

TGGGCTCAGGCTAGCTGAACAAGTTTCTAAATATGGTATTAAGGTATGTTGTGTT

GACCCTTCACCACTCTCCATGTGGCCAAATAATTATGGTGTTTGGGTTGATGAGT

TTGAGAATTTAGGACTGGAAAATTGTTTAGATCATAAATGGCCTATGACTTGTGT

GCATATAAATGATAACAAAACTAAGTATTTGGGAAGACCATATGGTAGAGTTAGT

AGAAAGAAGCTGAAGTTGAAATTGTTGAATAGTTGTGTTGAGAACAGAGTGAAGT

TTTATAAAGCTAAGGTTTGGAAAGTGGAACATGAAGAATTTGAGTCTTCAATTGT

TTGTGATGATGGTAAGAAGATAAGAGGTAGTTTGGTTGTGGATGCAAGTGGTTTT
GCTAGTGATTTTATAGAGTATGACAGGCCAAGAAACCATGGTTATCAAATTGCTC
ATGGGGTTTTAGTAGAAGTTGATAATCATCCATTTGATTTGGATAAAATGGTGCT
TATGGATTGGAGGGATTCTCATTTGGGTAATGAGCCATATTTAAGGGTGAATAAT
GCTAAAGAACCAACATTCTTGTATGCAATGCCATTTGATAGAGATTTGGTTTTCT
TGGAAGAGACTTCTTTGGTGAGTCGTCCTGTTTTATCGTATATGGAAGTAAAAAG
AAGGATGGTGGCAGGATTAAGGCATTTGGGGATCAAAGTGAAAAGTGTTATTGAG
GAAGAGAAATGTGTGATCCCTATGGGAGGACCACTTCCGCGGATTCCTCAAAATG
TTATGGCTATTGGTGGGAATTCAGGGATAGTTCATCCATCAACAGGGTACATGGT
GGCTAGGAGCATGGCTTTAGCACCAGTACTAGCTGAAGCCATCGTCGAGGGGCTT
GGCTCAACAAGAATGATAAGAGGGTCTCAACTTTACCATAGAGTTTGGAATGGTT
TGTGGCCTTTGGATAGAAGATGTGTTAGAGAATGTTATTCATTTGGGATGGAGAC
ATTGTTGAAGCTTGATTTGAAAGGGACTAGGAGATTGTTTGACGCTTTCTTTGAT
CTTGATCCTAAATACTGGCAAGGGTTCCTTTCTTCAAGATTGTCTGTCAAAGAAC
TTGGTTTACTCAGCTTGTGTCTTTTCGGACATGGCTCAAACATGACTAGGTTGGA
TATTGTTACAAAATGTCCTCTTCCTTTGGTTAGACTGATTGGCAATCTAGCAATA
GAGAGCCTTTGA (SEQ ID NO: 5).

More specifically, the nucleotide substitution A949G, caused the unusual presence of lycopene in the petals of the tomato plants and a reduction in β-xanthophylls, deriving from β-carotene, whereas it caused an accumulation of lycopene in the ripe fruit [30].

The presence of lycopene in the petals and the increase in lycopene in the fruit is explained by the deleterious effect that the missense mutation A949G of the gene *Cyc-B* has on the capacity of lycopene beta cyclase of converting lycopene into β-carotene, i.e. due to the amino-acid change, R317G, the cyclase effectively converts the lycopene into β-carotene with respect to the wild-type enzyme.

The increase in lycopene to the detriment of β-carotene was observed in the fruit of the mutant plants (mutant line *cyc-b7*) grown under both controlled conditions and in the open field.

### EXAMPLE 2: Comparative open field tests

### MATERIALS AND METHODS

### Breeding of the plants

In the open-field evaluation, the plants of the mutant line cyc-b7 having the mutation A949G in the gene Cyc-B were compared with the control line Red Setter and with the commercial tomato lines Dres F1 and Progress.

The seeds of the line cyc-b7 and Red Setter were first placed in polystyrene plateaux to germinate at a temperature of about 20-25°C and the plantlets at a 4-6 leaf stage were then transplanted in fields. The plantlets of Dres F1 and Progress, on the other hand, were purchased from a greenhouse.

The resistance capacity of the cyc-b7 genotype to the broomrape parasite was assessed for two consecutive years using plants of the generation M5 in the year 2013, and plants of the generation M6 in 2014.

The breeding of the plants cyc-b7, which were a total of 30, took place in an industrial tomato production field of the commercial variety Dres F1. The field tests were prepared in the area of Foggia.

The nature of cyc-b7 of resistance to the parasite was then evaluated by comparison with the non-mutagenized control line Red Setter and with Dres F1.

When the plants reached the ripening stage of the fruit, the presence of the infection was ascertained, i.e. the shoots were counted on a comparable number of plants belonging to the three genotypes being raised.

### RESULTS

### First open-field evaluation of the susceptibility of the line cyc-b7 to infection on the part of broomrape

The data obtained in the three measurements of the level of infection on the part of broomrape on the three genotypes of tomato Red Setter, cyc-b7, Dres F1 analyzed in the field test of the year 2013 are indicated in Table 2 below.

**TABLE 2**

| Genotype | Number of plants analyzed | Survey I 31-07-2013 | Survey II 07-08-2013 | Survey III 26-08-2013 |
|---|---|---|---|---|
| *Red Setter* | 7 | 27* | 35* | 8* |
| *cyc-b7* | 7 | 2* | 1* | 1* |
| *Dres* F1 | 16 | 134* | 66* | 26* |

| | | | | |
|---|---|---|---|---|
| * Number of broomrape shoots | | | | |

As can be observed, right from the first survey, the line cyc-b7, that has the allelic variant A949G of gene Cyc-B, showed a very low number of broomrape shoots with respect to the commercial line Dres F1 and the control line Red Setter.

The data obtained indicate that the mutant genotype is capable of resisting and opposing the parasite in conditions of high attack as can be seen from the significant number of shoots on the line Dres F1 in the first survey.

The results of this first trial, effected in the summer of 2013, even if produced on a very small number of plants, seem to confirm our hypothesis on the correlation between a reduction in the content of β-carotene and therefore strigolactone and a lower susceptibility to infection on the part of broomrape in the mutant cyc-b7.

### Second open-field evaluation of the susceptibility of the line cyc-b7 to infection on the part of broomrape

The data obtained in the pilot experiment of 2013 were extremely encouraging for continuing with the evaluation of the cyc-b7 genotype under attack conditions of the parasite.

A new field trial was in fact prepared in the period of May-August 2014, again in the area of Foggia where the problem of broomrape is strongly felt.

Plants of the generation M6 of the line cyc-b7 were compared with the Red Setter control plants and the commercial line Progress in a field for the industrial production of tomatoes (cv Progress).

In the trial of 2014, an extremely high number of cyc-b7 plants were used (about 100) in an experimental project with biological replicates.

When the plants reached the fruit-setting stage, surveys were effected on the presence of the parasite, more specifically, the shoots were counted on a comparable number of plants belonging to the three genotypes Red Setter, cyc-b7, Progress in cultivation. For each genotype, the presence of the parasite was ascertained on two plots (biological replicates), each consisting of 18 tomato plants. A total of three surveys were effected. Table 3 indicates the number of broomrape shoots per plot found on the three tomato genotypes in the field trial prepared in the year 2014. For each genotype, the survey was effected on two plots each consisting of 18 plants.

**TABLE 3**

| Genotype | Number of plants analyzed per plot | Survey I 02-07-2014 | Survey II 24-07-2014 | Survey III 05-08-2014 |
|---|---|---|---|---|
| *Red Setter* | 18 | 7* | 55* | 107* |
| | 18 | 19* | 185* | 89* |
| *cyc-b7* | 18 | 2* | 16* | 29* |
| | 18 | 0* | 8* | 12* |
| *Progress* | 18 | 16* | 153* | 105* |
| | 18 | 23* | 102* | 89* |

As can be observed in Table 3, in the first survey, the line cyc-b7, that has the allelic variant A949G of the gene Cyc-B, showed a very low number of broomrape shoots, specifically two in one plot and zero in the other, with respect to the commercial line Progress (16 and 23 shoots) and the control line Red Setter (7 and 19 shoots).

In the second survey, the total number of shoots in the two plots of cyc-b7 is still low and equal to about 10% of that of the control lines. 24 shoots are in fact registered in the mutant cyc-b7 against 240 and 255 respectively revealed for Red Setter and Progress. The difference in the number of shoots in the mutant line and two control genotypes also remains marked in the third survey of August 5, 2014. Cyc-b7, in fact, has an 80% reduction in the number of parasite plants with respect to Red Setter and Progress.

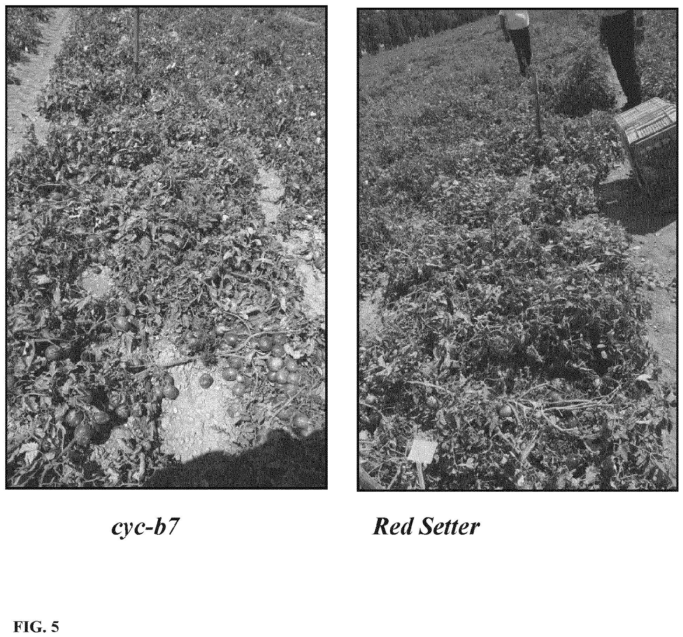

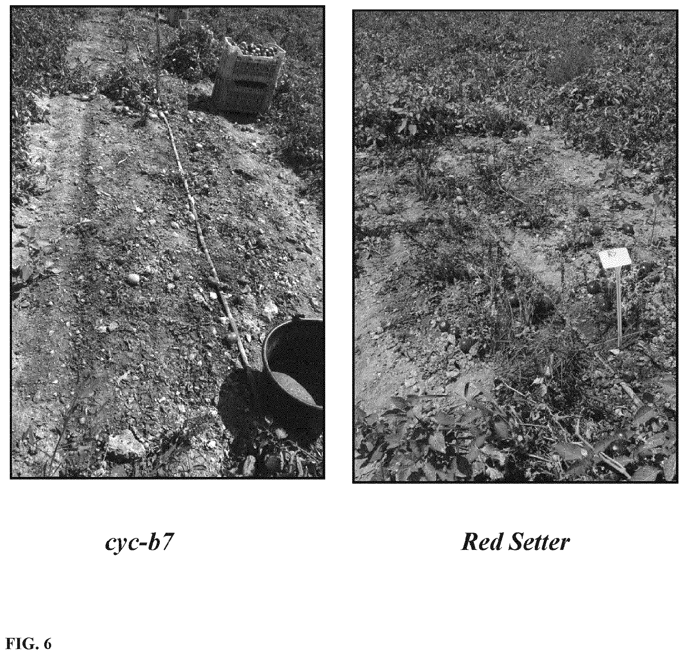

The difference in the number of shoots observed between the mutant line cyc-b7 and the Red Setter line is also documented by the photographs shown in Figures 5 and 6.

In Figure 5, the photographs show the presence of broomrape in a plot of the mutant line cyc-b7 and the Red Setter genotype before the harvesting of the tomato fruit. After the harvest and removal of the plants, in the photographs of Figure 6, the marked difference in the number of shoots present in the plot of the mutant line and that of the control line is much more evident.

From the data obtained, it can be asserted that the mutant genotype cyc-b7, in the presence of the attack of broomrape, has a good resistance to infection on the part of the parasite.

The resistance feature is not total but this is compatible with the type of mutation present in the allelic variant: a missense mutation that modulates and does not annul the activity of the protein as expected, for example, from a knock-out mutation.

The resistance phenotype observed in the mutant line cyc-b7 in the trial of 2013 is widely confirmed in the field trial of 2014 conducted with a higher number of mutant plants and with an experimental design of biological replicates.

The data produced in the two field trials show that the allelic variant A949G of the gene Cyc-B according to the invention can be used as a fighting instrument against infestations of broomrape in tomato plants. This can allow the production of tomatoes in areas currently affected by the attack of the parasite.

### Field trial of 2014: production data

In the field trial of 2014, in addition to a survey on the level of infestation of the parasite, data were collected on the production of the three genotypes in the presence of attack on the part of broomrape.

The production data for the mutant line cyc-b7 and the Red Setter and Progress control lines were taken on same plots analyzed for the presence of the broomrape parasite and are indicated in Table 4.

The data obtained for the mutant line cyc-b7 and the two control lines are the result of the average of 36 plants for each genotype.

**TABLE 4**

| Genotype | Production Kg/plant |
|---|---|
| *Red Setter* | 3.4 |
| *cyc-b7* | 3.9 |
| *Progress* | 3.5 |

As can be seen from an analysis of Table 4, the plant of the cyc-b7 genotype produced an average of 3.9 Kg of tomato fruit against the 3.4 and 3.5 Kg registered for Red Setter and Progress respectively.

The cyc-b7 mutant line having the allelic variant A949G of the gene Cyc-B, in the presence of broomrape, therefore produced more fruit with respect to the two control lines.

The difference observed in the production of the resistant genotype and control lines is not very high (about 10%) and can definitely be attributed to a delay in the attack of the parasite on the tomato plants registered in the season of 2014 in the field trial area.

The reduction in quantitative terms of the production of tomatoes very much depends on the vegetative development phase in which the plant is parasitized: if the attack takes place starting from the first growth stages of the tomato, the damage is obviously greater.

Furthermore, visibly, the architecture and morphology of the cyc-b7 plants proved to be unaltered with respect to those of the control plants, in particular with reference to the presence of lateral branchings.

The data indicated above show that the allelic variant A949G of Cyc-B therefore represents a precious genetic resource that can be used by breeders and varietal raisers as an instrument for fighting infestations of broomrape, overcoming the undesired effects on the development of the plant. As the mutation (allelic variant) is no more than an SNP, this can be used as molecular marker and therefore "followed", for facilitating and accelerating the insertion of the new feature also in other genetic backgrounds of tomatoes.

## Claims

1. Use of a nucleotide sequence encoding for the enzyme lycopene beta-cyclase Cyc-B **characterized by** the presence of the missense mutation in the position A949G, induced through mutagenesis to select a mutated tomato plant with a phenotype of resistance to infections by broomrapes, wherein said nucleotide sequence consists in SEQ ID NO:5.

2. Use of a nucleotide sequence according to claim 1, wherein said infection is by branched broomrape.

3. Screening method to identify a tomato plant resistant to broomrapes, said method comprising the analysis of a DNA sample from a tomato plant of interest for the presence of a nucleotide sequence encoding for the enzyme lycopene beta-cyclase Cyc-B **characterized by** the presence of the missense mutation in the position A949G consisting in SEQ ID NO:5.

## Patentansprüche

1. Verwendung einer Nukleotidsequenz, die für das Enzym Lycopin-Beta-Cyclase Cyc-B kodiert, **gekennzeichnet durch** das Vorhandensein der Missense-Mutation in der Position A949G, induziert durch Mutagenese, um eine mutierte Tomatenpflanze mit einem Phänotyp der Resistenz gegen Infektionen durch Sommerwurz zu selektieren, wobei die Nukleotidsequenz aus SEQ ID NO:5 besteht.

2. Verwendung einer Nukleotidsequenz nach Anspruch 1, wobei die Infektion durch verzweigten Sommerwurz erfolgt.

3. Screening-Verfahren zur Identifizierung einer Tomatenpflanze, die gegen Sommerwurz resistent ist, wobei das Verfahren die Analyse einer DNA-Probe aus einer Tomatenpflanze von Interesse auf das Vorhandensein einer Nukleotidsequenz umfasst, die für das Enzym Lycopin-beta-Cyclase Cyc-B kodiert, **gekennzeichnet durch** das Vorhandensein der Missense-Mutation in der Position A949G, bestehend aus SEQ ID NO:5.

## Revendications

1. Utilisation d'une séquence nucléotidique codante pour l'enzyme lycopène bêta-cyclase Cyc-B **caractérisée par** la présence de la mutation faux-sens en position A949G, induite par mutagenèse pour sélectionner un plant de tomate muté avec un phénotype de résistance aux infections par les orobanches, dans laquelle ladite séquence nucléotidique consiste en SEQ ID NO:5.

2. Utilisation d'une séquence nucléotidique selon la revendication 1, dans laquelle ladite infection est par l'orobanche rameuse.

3. Procédé de criblage pour identifier un plant de tomate résistant aux orobanches, ledit procédé comprenant l'analyse d'un échantillon d'ADN provenant d'un plant de tomate d'intérêt pour la présence d'une séquence nucléotidique codante pour l'enzyme lycopène bêta-cyclase Cyc-B **caractérisée par** la présence de la mutation faux-sens en position A949G consistant en SEQ ID NO:5.
